# EUROPEAN PATENT SPECIFICATION

(11) **EP 0 910 365 B1**
(45) Date of publication and mention of the grant of the patent: **25.06.2003**
(21) Application number: 97922668.5
(22) Date of filing: 15.04.1997
(51) Int. Cl.: A61K 31/33, A61K 31/35, A61K 31/40, A61K 31/445, A61K 31/47, A61K 31/54, A61K 41/00, A61P 35/00

(54) **GROWTH INHIBITION AND ERADICATION OF SOLID TUMORS USING A PROLACTIN MODULATOR AND A PHOTOSENSITIZER**
WACHSTUMSHEMMUNG UND ABTOETUNG SOLIDER TUMORE UNTER VERWENDUNG EINES PROLAKTIN-MODULATORS UND EINES PHOTOSENSITIZERS
INHIBITION DE LA CROISSANCE ET ERADICATION DE TUMEURS SOLIDES PAR L'UTILISATION D'UN MODULATEUR DE PROLACTINE ET D'UNE SUBSTANCE PHOTOSENSIBILISANTE

(30) Priority: 01.05.1996 US 16619 P
(43) Date of publication of application: 28.04.1999
(73) Proprietor: The General Hospital Corporation doing business as Massachusetts General Hospital, Boston, MA 02114 (US); PRESIDENT AND FELLOWS OF HARVARD COLLEGE, Cambridge Massachusetts 02138 (US)
(72) Inventor: CINCOTTA, Anthony, H., Charlestown, MA 02129 (US); CINCOTTA, Louis, Andover, MA 01810 (US)
(74) Representative: Grünecker, Kinkeldey, Stockmair & Schwanhäusser Anwaltssozietät
(86) International application number: US9707577
(87) International publication number: WO97040829

(56) References cited:
- US-A- 5 225 433
- US-A- 5 468 755
- DATABASE CHEMABS [Online] CHEMICAL ABSTRACTS SERVICE, COLUMBUS, OHIO, US COPPOLA, ANTONIO ET AL: "Augmentation of photodynamic effects: the synergistic action o hematoporphyrin and chlorpromazine" retrieved from STN Database accession no. 106:134351 XP002117596 & J. TUMOR MARKER ONCOL. (1986), 1(3), 205-11 ,
- DATABASE CHEMABS [Online] CHEMICAL ABSTRACTS SERVICE, COLUMBUS, OHIO, US CINCOTTA, L. ET AL: "Novel red absorbing benzo[a]phenoxazinium and benzo[a] phenothiazinium photosensitizers: in vitro evaluation" retrieved from STN Database accession no. 108:91067 XP002117597 & PHOTOCHEM. PHOTOBIOL. (1987), 46(5), 751-8 ,
- CINCOTTA, LOUIS ET AL: "Phototoxicity, redox behavior, and pharmacokinetics of benzophenoxazine analogs in EMT-6 murine sarcoma cells" CANCER RES. (1993), 53(11), 2571-80 , XP002117595

## Description

### BACKGROUND OF THE INVENTION

### Field of the Invention

Disclosed herein is the use of a prolactin modulator and a photosensitizer for the preparation of a medicament for the treatment of a mammal bearing one or more tumors.

### Treatment of Tumors

It is well known in the art to inhibit the growth of tumors by using cytotoxic compositions or ionizing radiation. A major drawback of ionizing radiation as a therapeutic modality is that it often results in injury or damage to healthy tissue in the vicinity of, or in contact with, the malignant tumor cells. Cytotoxic compositions have the even greater drawback of often causing systemic toxicity, i.e. damaging tissues at loci distal to the tumor.

One known method for killing or treating tumor cells is by contacting them with a photosensitizer substance and thereafter exposing the contacted cells to light of a predetermined wavelength (Kessel. D., *International Photodynamics* March 1995, pp. 2-3; Dougherty, T.J. et al., *Photochem. Photobiol.* 45:879-89, 1987; Moan, J. et al., *Photochem. Photobiol.* 55:145-57, 1992). This so-called photodynamic therapy (PDT) for treatment of tumors selectively eradicates tumor tissue without the deleterious side effects that are often seen when ionizing radiation or chemotherapy is employed. However, the two most widely studied PDT drugs, hematoporphyrin (HPD) and Photofrin II, suffer from several limitations such as:
(i) both exhibit a low absorption coefficient in the region where light penetrates tissue most efficiently (600-800 nm);
(ii) both are complex mixtures of porphyrin ethers and ester oligomers;
(iii) prolonged retention of these photosensitizers in the skin leads to dermal photosensitization that can persist for months.

It has now been unexpectedly discovered that the efficacy of photodynamic therapy for arresting the growth of or eradicating tumors can be significantly enhanced by normalizing the prolactin and/or melatonin profiles of the mammal receiving such treatment to approach or conform to the respective profiles of a young, healthy mammal of the same sex and species.

### Prolactin and Neuroendocrine Rhythms

Research has demonstrated that circadian rhythms play important roles in regulating prolactin activities and vice versa.

Publications such as Meier, A.H., Gen. Comp. Endocrinol. 3(Suppl 1):488-508, 1972; Meier, A.H., Trans. Am. Fish. Soc. 113:422-431, 1984; Meier, A.H. et al., Current Ornithology II (ed Johnston R.E.) 303-343, 1984; Cincotta, A.H. et al., J. Endocrinol. 120:385-391, 1989; Meier, A.H., Amer. Zool. 15:905-916, 1975; Meier, A.H., Hormonal Correlates of Behavior (eds. Eleftherton and Sprott) 469-549, 1975 disclose how circadian rhythms regulate prolactin activities. The resulting daily variations in responsiveness of different cell types to prolactin have a primary role in regulating numerous physiological processes, including fat storage, lipogenic responsiveness to insulin, migratory behavior, metamorphosis, reproduction, growth, pigeon cropsac development and mammary development (Meier, A.H., Gen. Comp. Endocrinol. 3(Suppl 1):488-508, 1972; Meier, A.H., Amer. Zool. 15:905-916, 1975; Meier, A.H. et al., Science 173:1240-1242, 1971). In regulating one of the foregoing physiological activities, prolactin may be observed to produce a stimulatory or an inhibitory effect on a given activity, or to have no effect on it. These varying effects have recently been shown in animals to be a function of the time of the daily endogenous peak (i.e. acrophase) of the rhythm of plasma prolactin concentration or a function of the time of daily injection of exogenous hormone (or of a substance that increases prolactin levels) or of the relation between endogenous peak and any induced peak. Furthermore, high levels of prolactin restricted to a discrete daily interval have a much greater physiologic (e.g. metabolic) effect in animals than do constant high levels throughout a day (Cincotta, A.H. et al., Horm. Metab. Res. 21:64-68, 1989; Borer, K.T. in The Hamster: Reproduction and Behavior (ed. Siegel, H.I.) 363-408, 1985). Such findings demonstrate the existence of daily response rhythms to prolactin by certain types of cells.

One early demonstration of a daily variation in physiological responsiveness to any hormone was the dramatic variation in fattening responsiveness to prolactin in the white-throated sparrow (Meier, A.H. et al., Gen. Comp. Endocrinol. 8:110-114, 1967). Injections at midday of a 16-hour daily photoperiod stimulated 3-fold increases in body fat levels, whereas injections given early in the photoperiod reduced fat stores by 50%. Such daily variations in fattening responses to prolactin have subsequently been demonstrated in numerous species of all the major vertebrate classes (Meier, A.H., Amer. Zool. 15:905-916, 1975; Meier, A.H., Hormonal Correlates of Behavior (eds. Eleftherton and Sprott) 469-549, 1975) indicating the fundamental nature of such a temporal organization. The fattening response rhythm persists under constant light conditions (Meier, A.H. et al., Proc. Soc. Exp. Biol. Med. 137:408-415, 1971) indicating that it, like many other endogenous daily variations, is a circadian rhythm.

Additional studies have demonstrated that circadian rhythms have primary roles in regulating numerous physiologic activities, such as immune function, lipid metabolism, and body fat stores (Cincotta, A.H. et al., Endocrinology 136(5):2163-2171, 1995; Meier, A.H. et al., Current Ornithology II (ed Johnston R.E.) 303-343, 1984; Meier, A.H., Amer. Zool. 15:905-916, 1975; Meier, A.H., Hormonal Correlates of Behavior (eds. Eleftherton and Sprott) 469-549, 1975; Meier, A.H. et al., J. Am. Zool. 16:649-659, 1976); Cincotta et al., Life Sciences 45:2247-2254, 1989; Cincotta et al., Ann. Nutr. Metab. 33:305-14, 1989; and Cincotta et al., Horm. Metabol. Res. 21:64-68. 1989.

The immune function studies (Cincotta, A.H. et al., Endocrinology 136(5):2163-2171, 1995) showed that the responsiveness of immune system components to prolactin is time of day dependent. Timed daily administrations of prolactin or bromocriptine were shown to be able to stimulate or inhibit immune responses, depending on the time of day that they are administered. That is, there was found to be a specific window of time during which prolactin was found to have an immunostimulatory effect, outside of which prolactin exerted no effect. Conversely, there was found to be a specific window of time during which bromocriptine, a prolactin inhibitor, was found to have an immunosuppressive effect, outside of which it had no effect on immune function. These findings indicate an essential role for prolactin rhythms in the regulation of immunity.

The experiments relating to metabolism showed that an interaction of circadian rhythms of liporegulatory hormones (stimuli) and of circadian responses to these hormones (in target cells) determines amount of lipogenesis and fat storage. Thus, high plasma concentrations of prolactin (which serves as the stimulus) occur during the daily interval of maximal fattening responsiveness to prolactin in fat animals, but occur at other unresponsive times of day in lean animals (Meier, A.H., Amer. Zool. 15:905-916, 1975; Meier, A.H., Hormonal Correlates of Behavior (eds. Eleftherton and Sprott) 469-549, 1975; Speiler, R.E. et al., Nature 271:469-471, 1978). Similarly, plasma insulin (which acts as the stimulus) levels are highest during the daily interval of greatest hepatic lipogenic response to insulin in obese hamsters, but at a different time of day in lean hamsters (deSouza, C.J. et al., Chronobiol. Int. 4:141-151, 1987; Cincotta, A.H. et al., J. Endocr. 103:141-146, 1984). The phase relationships of these stimulus and response rhythms are believed to be expressions of neural circadian centers which in turn can be reset by neurotransmitter agents and hormone injections (including prolactin) to produce either fat or lean animals (Meier, A.H., Trans. Am. Fish. Soc. 113:422-431, 1984; Meier, A.H. et al., Current Ornithology II (ed Johnston R.E.) 303-343, 1984; Cincotta, A.H. et al., J. Endocrinol. 120:385-391, 1989; Emata, A.C. et al., J. Exp. Zool. 233:29-34, 1985; Cincotta, A.H. et al., Chronobiol. Int'1 10:244-258, 1993; Miller, L.J. et al., J. Interdisc. Cycles Res. 14:85-94, 1983). Accordingly, timed prolactin administration or enhancement has been shown to act directly upon tissues (e.g. liver in lipogenesis) undergoing circadian rhythms of responsiveness to the hormone to produce immediate variations in net physiologic effects (Cincotta, A.H. et al., Horm. Metab. Res. 21:64-68, 1989) and also acts indirectly by resetting one of the circadian neuroendocrine oscillations of a multi-oscillatory circadian pacemaker system to establish different phase relations between the multiple circadian (neural, hormonal, and tissue) expressions that control lipid metabolism (Meier, A.H., Trans. Am. Fish. Soc. 113:422-431, 1984; Meier, A.H. et al., Current Ornithology II (ed Johnston R.E.) 303-343, 1984; Cincotta, A.H. et al., J. Endocrinol. 120:385-391, 1989; Emata, A.C. et al., J. Exp. Zool. 233:29-34, 1985; Cincotta, A.H. et al., Chronobiol. Int'l 10:244-258, 1993; Miller, L.J. et al., J. Interdisc. Cycles Res. 14:85-94, 1983).

It has previously been shown that prolactin, or substances that affect circulating prolactin levels, also affect circadian rhythms and in fact can be used to modify such rhythms (so that they more closely resemble the rhythms of lean, healthy, young individuals of the same sex) and to reset such rhythms (so that the modified rhythms persist in the modified condition). See, e.g. U.S. Patent Applications 08/158,153, 07/995,292, 07/719,745, 07/999,685 08/171,569, and U.S. Patent No. 5,344,832. This prior work by the present inventors has been clinically tested in humans afflicted with various physiological disorders (obesity, diabetes, atherosclerosis, hypertension, immune dysfunction, and others) with meaningful results.

In particular, in U.S. Patent Application Serial No. 071995,292 (now allowed), and in its continuation-in-part Serial No. 08/264,558, filed June 23, 1994, the present inventors disclose a method for the reduction in a subject, vertebrate animal or human, of body fat stores, and reduction of at least one of insulin resistance, hyperinsulinemia, and hyperglycemia, and other metabolic diseases, especially those associated with Type II diabetes. More specifically, the foregoing applications disclose methods for: (i) assessing the daily prolactin level cycles of a normal (healthy) human or vertebrate animal (free of obesity, disease or other disorder); (ii) diagnosing aberrant daily prolactin level cycles of a human or vertebrate animal; and (iii) determining the appropriate adjustments that need to be made to normalize such aberrant prolactin level cycles. This method involves the administration of at least one of a prolactin reducer and/or a prolactin enhancer at a first predetermined time (or times) within a 24-hour period (if only a prolactin reducer is administered) and/or at a second predetermined time (or times) of a 24-hour period (if a prolactin enhancer is administered). This therapy. when continued for several days, weeks or months, results in the long-term adjustment of aberrant or abnormal prolactin level cycles so that they conform to (or approach) normal prolactin level cycles. In most cases, this benefit persists over the long-term even after cessation of therapy. As a result, aberrant physiological parameters associated with various metabolic disorders are restored to normal levels or are modified to approach normal levels.

Further illustration of the utility of resetting prolactin rhythms can be found in U.S. Patent Application Serial No. 08/271,881, filed July 7, 1994, a method for regulating immune function by resetting prolactin rhythms is disclosed, and in U.S. Patent Application Serial No. 08/475,296 filed June 7, 1995, a method for arresting the growth of or eradicating neoplastic growths in mammals having daily prolactin rhythms is disclosed.

### Melatonin

Other studies have shown that melatonin levels tend to be altered in humans suffering from tumors (Bartsch, C. et al. Ann. N.Y. Acad. Sci. 719:502-525, 1994) and that sera from tumor-bearing animals can suppress melatonin secretion by pineal organ cultures (Leone, A.M. et al., J. Pineal Res. 17:17-19, 1994). Thus, while other studies have correlated tumors with abnormal melatonin levels and secretion, there is no teaching in the prior art of the desirability of adjusting abnormal melatonin daily rhythms in mammals in order to inhibit or destroy tumors.

### PHOTODYNAMIC THERAPY (PDT)

PDT is a promising new approach for the selective eradication of tumors which does not result in the deleterious side effects that are often experienced with both chemotherapy and ionizing radiation therapy. PDT involves the systemic administration of tumor localizing photosensitizers that can kill malignant tissue when irradiated with light of the appropriate wavelength. Photoactivation of photosensitizers in the presence of oxygen generates highly reactive and cytotoxic molecular species by one or both of the following mechanisms:
(a) a type I reaction where the excited state of the dye interacts directly with biomolecules to generate free radicals, hydrogen peroxide, superoxide, etc.; or
(b) a type II reaction where the excited state of the dye interacts directly with oxygen to generate the highly reactive, short-lived cytotoxin singlet oxygen (¹O₂).

*In vitro* these oxidizing species cause cell death as a result of damage to various cellular organelles and functions depending on the photosensitizer used. *In vivo,* however, studies of the acute effects of PDT on animal tumors using a variety of sensitizers have shown that vascular occlusion is largely responsible for tumor eradication. This treatment modality has progressed to phase III clinical trials using two PDT drugs, hematoporphyrin (HPD) and Photofrin II (PII). Although encouraging results have been obtained with these PDT agents for a wide variety of tumors, it has become apparent that in order to realize the full potential of PDT additional sensitizers needed to be developed. The reported limitations of both HPD and PII include:
(a) a low absorption coefficient in the region where activating light penetrates tissue most efficiently (600-900 nm);
(b) both products are not a single entity but consist of mixtures of porphyrin ether and ester oligomers;
(c) prolonged retention in the skin leads to dermal photosensitization that can persist for months; and finally
(d) the rapid formation of hypoxic cells which occurs as a consequence of the vasculature damage during PDT with these photosensitizers increases the probability that a fraction of the tumor cells will escape direct photodestruction. Nutritional resupply to these still viable tumor cells through diffusion or angiogenesis may rapidly repopulate the tumor (Henderson et al., *Photochem. Photobiol.* 49:299-304, 1989; Henderson et al., *Cancer Res.* 47:3110-3114, 1987).

The success of PDT and the limitations of HPD and PII have stimulated the search for more efficacious phototoxic compounds primarily within the porphyrin family (i.e. benzoporphyrins, chlorins, purpurins, phthalocyanines, etc.); thus these second generation drugs tend to have similar PDT properties, i.e., there is not a great deal of differentiation in the accumulation of the photosensitizers in normal vs. tumor cells, or in mode of cell killing, (i.e. vascular occlusion as for HPD and Photofrin II). An approach of the present inventors has been to study diverse classes of photosensitizers which possess intrinsically different physicochemical and pharmacological properties and to synthesize a large number of novel photosensitizing chromophores for PDT (described in Foley et al., U.S. Patent No. 4,962,197).

*In vitro* investigations have established that the cyanine type, phthalocyanine type, porphyrin type, and benzophenoxazine analog dyes of the present invention possess characteristics which allow for their beneficial use in the treatment of tumors (Richter, A.M. et al., J. Nat. Cancer Inst., 79:1327-1332, 1987; Foultier, M-T., et al. J. Photochem. Photobiol.B:Biol., 10:119-132,1991; Morgan, A.M., et al. *Future Directions and Applications in Photochemistry and Photobiology,* SPIE Institute Series, Vol. IS 6:87-106, 1990). These include: (a) a high degree of lipophilicity; (b) rapid tumor localization; (c) absorption of light in a spectral region where light penetrates tissue maximally; and (d) efficient generators of phototoxin. The benzophenoxazine analogs seem to rapidly accumulate intracellularly and cause tumor destruction with minimal damage to the vasculature, unlike HPD or Photofrin II. This is believed to potentiate the effect of the therapy because oxygen supplied to tumor cells is required for the cytotoxic effects of PDT including benzophenoxazine analogs (Cincotta et al., Cancer Res. 54:1249-1258, 1994; Lin, C-W. et al., Cancer Res. 51:1109-1116, 1991; Foster, T.H., et al., Radiation Res, 126:296-303, 1991; Foster, T.H. et al., SPIE Proc. 1645:104-114, 1992).

US 5,225,433 relates to the diagnosis and treatment of abnormal and neoplastic tissues such as tumors by the single use of photosensitizing drugs, that accumulate in the tissue.

Cincotta *et al.* (Cancer Research **53**, 2571-5580, June 1, 1993) relates to the phototoxity, redox behavior and pharmacokinetics of benzophenoxazine analogues *in vitro* such as 5-ethyl-9-diethylaminobenzo[a]phenoxazinium in EMT-6 murine sarcoma cells. Cincotta *et al.* (Photochem. Photobiol. **46** (5), 751-8, 1987) relates to the phototoxity of benzophenoxazine analogues *in vitro* such as 5-amino-6-iodo-9-diethylaminobenzo [a] phenoxazinium in a murine sarcoma 180 and 4 human carcinoma cell lines.

Coppola *et al.* describes the cytotoxity of chloropromazine (CPZ) in combination with hematoporphyrin derivatives (HP-D) and cold white light to mouse erythrocyte and C3HST4 lymphoma cells. Neither CPZ nor HP-D was toxic alone.

US 5,468755 relates to a therepeutic process for the treatment the pathologies of type II diabetes by the single administration of bromocriptin to a vertebrate.

### SUMMARY OF THE INVENTION

It has long been known that mammals (including humans) suffering from tumors have abnormal prolactin profiles. It has been known for quite some time that melatonin levels are also abnormal in mammals suffering from tumors. As disclosed in copending U.S Patent application Serial No. 08/475,296 filed June 7, 1995, it has recently been unexpectedly discovered that the growth of tumors in mammals (including humans) may be treated by modifying the abnormal prolactin profile of the mammal afflicted with tumors so that the profile approaches or conforms to the prolactin profile of a lean, young healthy mammal of the same species and sex (the normal profile). It was shown that the abnormal prolactin profile of the afflicted mammal may be modified by:
(i) direct administration of prolactin,
(ii) adjusting the prolactin profile by timed administration of prolactin modulators, i.e. prolactin enhancers and/or reducers, or by
(iii) resetting the circadian rhythm of the afflicted mammal to a normal phase and amplitude through the timed administration of prolactin enhancers and prolactin reducers (such as bromocriptine).

It has also been known that PDT is a promising method of treating tumors without the severe side effects of standard chemotherapy and ionizing radiation.

It has now been surprisingly and unexpectedly discovered that the growth arrest or eradication of tumors that can be achieved with PDT can be significantly augmented by normalizing one or both of the prolactin and melatonin profiles of the tumor bearing mammal to the respective normal profiles for a mammal of the same species and sex. This discovery was entirely unexpected because (a) the mechanism of action of PDT is completely unrelated to and independent of the mechanism for adjustment of prolactin and melatonin daily rhythms, and (b) there has been no previous report of treating tumors in mammals by normalizing melatonin daily rhythms.

The photodynamic therapy is used to treat solid neoplastic tumors including by way of non-limiting example sarcomas, carcinomas. and gliomas. Among the specific neoplastic tumors that have been successfully treated (i.e. reduced in size or eliminated entirely) using PDT are papillary bladder tumors, lung cancer, obstructing esophogeal tumors, gastric, colon, and cervical cancers. Metastatic breast cancer tumors can also be treated as well as squamous cell and basal cell skin cancers. The tumor to be treated must be accessible to a source of actinic light. Thus the treatment can be practiced on surface lesions. Tumors in internal organs may be treated using, for example, fiber optic devices to enable exposure of the tumors to actinic radiation. The tumors referred to in this specification are all malignant tumors.

One embodiment of the present invention is the use of a prolactin modulator and a photosensitizer for the preparation of a medicament for the simultaneous or sequential use of said prolactin modulator and said photosensitizer for treating a mammal bearing one or more tumors.

Another embodiment of the present invention is the use of a prolactin modulator for the preparation of a medicament or a pharmaceutical composition for the simultaneous or sequential use of said medicament or pharmaceutical composition with a photosensitizer for treating a mammal bearing one or more tumors.

A further embodiment of the present invention is the use of a photosensitizer for the preparation of a medicament or a pharmaceutical composition for the simultaneous or sequential use of said medicament or pharmaceutical composition with a prolactin modulator for treating a mammal bearing one or more tumors.

A preferred embodiment of the present invention comprises a use as explained above, wherein said prolactin modulator is a prolactin enhancer. Another preferred embodiment comprises a use as explained above, wherein said prolactin modulator is a prolactin reducer. In a more preferred embodiment, said prolactin enhancer is administered to said tumor bearing mammal at a predetermined time or times to increase the night time prolactin levels of said mammal so that said mammal's night time prolactin level conforms to or approaches the normal night time prolactin profile. In a most preferred embodiment, said predetermined time is night time.

In another preferred embodiment according to a use of the present invention said prolactin enhancer is a member selected from the group consisting of prolactin, melatonin, metoclopramide, domperidone and 5-hydroxytryptophan. Also preferred is a use as explained above, wherein said prolactin enhancer is melatonin and said melatonin is administered in an amount within the range of 0.5-20 mg/person/day.

Further, the present invention provides a use as explained above, wherein said prolactin reducer is administered to said tumor bearing mammal at a predetermined time or times to decrease the day time prolactin levels of said mammal so that said mammal's day time prolactin level conforms to or approaches the normal day time prolactin profile. Preferably, said predetermined time is between about 6:00h and 10:00h.

Also preferred is a use as explained above, wherein said prolactin reducer is a dopamine agonist. More preferably said prolactin reducer is bromocriptine and said bromocriptine is administered in an amount within the range of 0.8 to 4.8 mg/person/day.

Further, the invention provides a use as explained above, wherein said tumor bearing mammal is a human. The invention also provides a use as explained above, wherein said photosensitizer is selected from the group consisting of porphyrin dyes, phtalocyanin dyes, cyanine dyes, benzophenoxazine analogs, and pharmaceutically acceptable salts thereof. Preferably, said photosensitizer is a benzophenothiazine. More preferably said benzophenothiazine is a member selected from the group consisting of 5-ethylamino-9-diethylamino-benzo[a]phenothiazinium and 5-ethylamino-9-diethylamino-benzo-2-iodophenothiazinium, or a pharmaceutically acceptable salt thereof or a member selected from the group consisting of 5-ethylamino-9-diethylamino-benzo[a]phenothiazinium chloride and 5-ethylamino-9-diethylamino-benzo-2-iodophenothiazinium chloride (Dye 4-115).

Thus, the present invention is directed to the use of a prolactin modulator and a photosensitizer for the preparation of a medicament for treating or inhibiting the growth of tumors in mammals by adjusting the circadian rhythms of prolactin and melatonin with said medicament.

Advantages of the present invention include:
- enhanced reduction in tumor growth and accelerated eradication of tumors.
- the ability to inhibit or eradicate malignant tumors without the debilitative effects of chemotherapeutic agents or ionizing radiation.
- the tumor growth inhibiting and treatment benefits of the medicament according to the present invention may persist long-term even after the administration of prolactin, melatonin, and PDT have been discontinued.

Other features and advantages of the present invention will be apparent from the following description taken in conjunction with the accompanying drawings.

### BRIEF DESCRIPTION OF THE DRAWINGS

Figure 1 depicts the nórmal or baseline prolactin profile for healthy male and female humans.
Figure 2 is the prolactin daily rhythm or profile curve for breast cancer patients with tumors.
Figure 3 is the normal melatonin daily rhythm or baseline melatonin profile for healthy male humans.
Figure 4 is the melatonin daily rhythm or profile curve for men with prostate cancer.
Figure 5 is a bar graph illustrating the effects of prolactin adjustment therapy alone, PDT alone, and the combination of prolactin adjustment and photodynamic therapies on tumor size in the EMT-6 implanted tumor mouse model system.
Figure 6 shows representative porphyrin photosensitizers (BPD-MA, Mono-L-Aspartyl Chlorin e6. Tin etiopurpurin) and phthalocyanine photosensitizers (aluminum, zinc, and silicon pthalocyanine).
Figure 7 shows representative benzophenoxazine analog photosensitizers (EtNBS, Dye 4-115), sulfated phthalocyanine, and cyanine photosensitizers (EDKC, pyrilium dyes, merocyanine 540).

### DETAILED DESCRIPTION OF THE INVENTION

As used in this specification, the following terms are intended to have the meanings set forth below.

Benzophenoxazine analogs include benzophenoxazines, benzophenothiazines, and benzophenoselenazines.

The terms "dye", "photosensitizer", and "chromophore" are used interchangeably.

"Prolactin reducer" refers to a substance or composition that has the ability to lower circulating prolactin levels upon administration to a mammal; "Prolactin enhancer" refers to a substance or composition that has the ability to raise circulating prolactin levels upon administration to a mammal, and includes prolactin itself.

Prolactin reducers and prolactin enhancers are referred to collectively as "prolactin modulators".

"Prolactin profile" of a subject is a depiction of circulating prolactin levels and their variation over all or part of a 24 hour period, and therefore is expression of all or part of the subject's plasma prolactin daily rhythm.

"Melatonin profile" of a subject is a depiction of circulating melatonin levels and their variation over all or part of a 24 hour period, and therefore is expression of all or part of the subject's plasma melatonin daily rhythm.

"Hormonal rhythms" include, but are not limited to, the variation over all or part of a 24 hour period of blood levels of prolactin and melatonin.

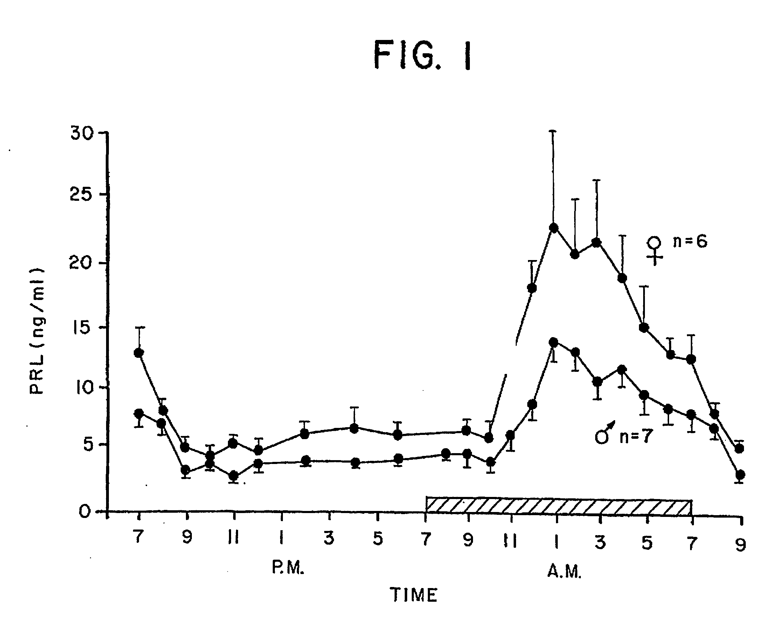

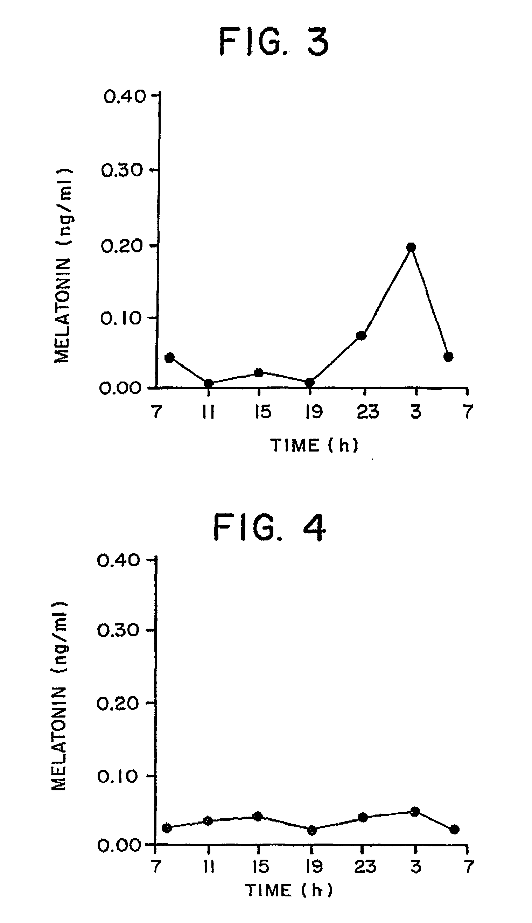

The term "healthy" refers to a young, lean subject free of disease including malignancies, tumors, immune system dysfunctions and metabolic abnormalities. A healthy subject is one with normal prolactin and melatonin profiles, i.e., profiles that does not depart from the baseline of that subject's species and sex by more than one standard error of the mean (SEM). The normal or baseline prolactin profile for healthy male and female humans is depicted in Figure 1. The normal or baseline melatonin profile for healthy male humans is depicted in Figure 3.

### DETERMINATION AND ADJUSTMENT OF PROLACTIN RHYTHMS

In order to avoid "false positives" a subject will not generally be considered to have an abnormal prolactin profile unless:
(a) the subject's daytime blood prolactin level is at least 1 standard error of the mean (SEM) higher than the baseline at two (or more) time points during daytime spaced apart by at least one and preferably by at least two hours; or
(b) the subject's daytime blood prolactin level is at least 2 SEM higher than the baseline at one time point during daytime; or
(c) the subject's night time blood prolactin level is at least 1 SEM below the base line at two (or more) time points spaced apart (as in (a)); or
(d) the subject's night time blood prolactin level is at least 2 SEM below the base line at one time point during night time.

The human male and female prolactin baselines are depicted in Fig. 1. One SEM during waking hours (07:00 - 22:00) is about 1-2 ng/ml for males and about 1-3 ng/ml for females; one SEM during night time (22:00 - 07:00) is about 3 ng/ml for males and about 3-6 ng/ml for females.

The characteristics of the prolactin level daily rhythm or profile that are to be approached or conformed to in humans include achieving low prolactin levels (2-7 ng/ml of plasma) for males and 2-10 ng/ml for females) during most or all of the time period between 07:00 and 22:00 h.

Ideally, a peak prolactin level in humans should also be achieved between the hours of 22:00 and 07:00 (preferably between 1:00 and 4:00) (the peak should be at least 10 ng/ml and most preferably between 10-15 ng/ml for males and at least 15 ng/ml and preferably between 15 and 25 ng/ml for females).

### Effects of Prolactin Modulators on Solid Tumors

The present invention provides uses of a prolactin modulator and a photosensitizer for the preparation of a medicament for treating and inhibiting the growth of tumors in mammals having a tumor burden and a prolactin daily rhythm.

The medicament prepared by the use according to the present invention may be used to:
(a) adjust the prolactin profile of the mammal to conform to or approach the normal prolactin profile for healthy members of the same species and sex as said mammal;
(b) the cells of said tumor are then contacted with a photosensitizer and exposed to light of a predetermined wavelength, power density, and energy level.

Thus, one aspect of this treatment is the administration of a medicament comprising a prolactin modulator(s) to the tumor bearing mammal at a predetermined time(s) during a 24-hour period. The time for administration of the prolactin modulator is selected so as to adjust the prolactin profile of the mammal receiving treatment to conform or approach the prolactin profile of a healthy mammal of the same sex and species.

It has been found that administration of prolactin enhancers is inhibitory to tumor growth in mammals when given at timed intervals during a 24 hour period which correspond to the peak of prolactin secretion in healthy mammals. Timed prolactin injections in tumor bearing mice which have had their circadian rhythms synchronized with a defined photoperiod were shown to exhibit a decreased tumor burden as compared with tumor bearing mice which did not receive timed prolactin injections. It has also been found that the effect of in vivo prolactin modulation on in vivo tumor growth is time-of-day dependent.

A time-of-day dependent role for prolactin in inhibiting tumors is found when experiments are performed on mice which decrease prolactin blood levels (by administration of a prolactin reducer) during specific daily intervals of lack of tumor growth inhibitory response to exogenous prolactin. Administration of bromocriptine, a D2 dopamine agonist which inhibits endogenous prolactin secretion, increases the inhibition of tumor growth when it is administered at times during a 24 hour period predetermined to reduce prolactin levels to those found in healthy animals of the same sex and species during such time period. The use of bromocriptine for inhibiting tumor growth is shown in Example 4.

The use of melatonin for inhibiting tumor growth is shown in Example 5. In this example the melatonin blood levels of mice are increased by the administration of melatonin, at a predetermined time known to be the interval of increased responsiveness to melatonin. It is found that the administration of melatonin at the time during a 24 hour period when melatonin levels are peaking in healthy mice exerts a potent inhibitory effect on growth of tumors.

These examples establish the ability of prolactin and melatonin to modulate tumor growth, and the relationship between tumor growth inhibition, endogenous prolactin levels (or prolactin enhancers or reducers), and the time of day of prolactin reduction or enhancement.

Although the foregoing experiments are conducted in mice, they are dependent on features of physiology that are common to mammals having a prolactin daily rhythm including humans. These experiments demonstrate that blood levels of prolactin and melatonin can be manipulated during predetermined intervals to bring about a desirable result with regard to inhibition of growth of tumors.

The alteration of prolactin the inhibition of tumor and metastases growth in a subject levels of a subject at particular times of day using the medicament prepared according to the present invention will allow. The medicament prepared according to the present invention may be used on all types of tumors, including but not limited to sarcomas, carcinomas, glioblastomas, melanomas, basal and squamous cell carcinomas, lymphomas, adenomas, and leukemias.

It is known that young adult healthy mammals of a given species (and sex), e.g. humans (suffering from no hormonal or metabolic disorders or cancer or other infection or ailment) have highly predictable daily prolactin level rhythms or profiles. The baseline curve for healthy human males and females in Figure 1 is derived from such young healthy individuals.

The phase relationship between the daily peaks of the stimulus (plasma prolactin) rhythm and response (tumor growth inhibition) to prolactin has been found to be important in tumor growth inhibitory activity. Environmental and pharmaceutical factors influencing either of these rhythms can be expected to impact tumor growth.

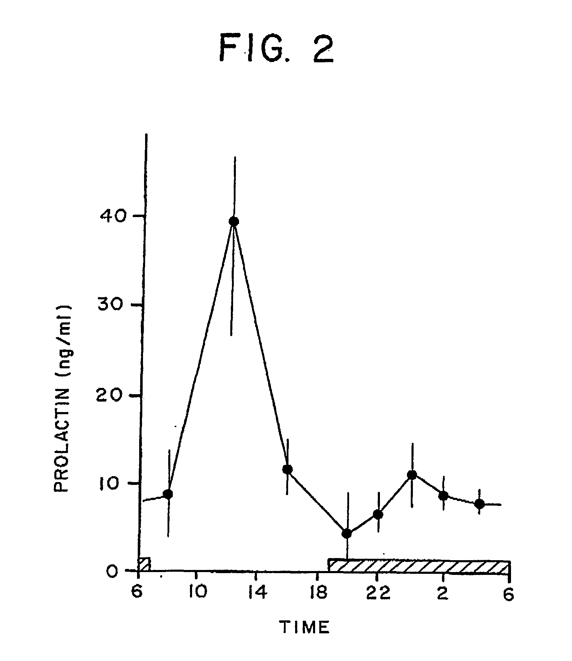

Humans with solid tumors, such as found in breast cancer and prostate cancer, have perturbed prolactin rhythms, which is apparent in a comparison of the prolactin rhythms of healthy women with the rhythms of women with breast cancer, which rhythms are shown in Figures 1 and 2, respectively. Humans with tumors thus may be able to benefit to a significant extent by adjustment of their prolactin daily rhythms (as expressed by their prolactin profile) to conform to or approach the normal or baseline prolactin curve of Figure 1. An adjusted prolactin profile approaches a normal or healthy profile, if all or a portion of the abnormal profile moves in the correct direction by at least 2 ng/ml.

One approach to adjusting prolactin profiles in a subject is as follows:
(i) the prolactin levels of the tumor bearing human should be ascertained by assaying blood samples of the tumor bearing human at certain spaced apart intervals within a 24 hour period (or portions thereof), and
(ii) the resultant prolactin profile of the tumor bearing human should be compared to the prolactin profile for a healthy human of the same sex.

Depending on the difference between (i) and (ii), the adjustment then involves administering a medicament comprising one or both of the following:
(a) a prolactin reducer at a first predetermined time (or at more than one first predetermined time) and in a first amount effective to reduce day time prolactin levels if these levels are too high; and
(b) a prolactin enhancer at a second predetermined time (or at a plurality of second predetermined times) and in a second amount effective to increase night time prolactin levels if these levels are too low.

In general, if a medicament comprising a prolactin lever altering substance is to be administered, appropriate allowance should be made with respect to the time of administration to permit that substance (depending on its pharmacokinetic properties) to affect prolactin levels such that prolactin levels would be modified during the appropriate time of day. Thus, the prolactin altering substance will be administered as follows:
(a) if prolactin is administered, it will be administered, preferably by injection, during the time interval that prolactin levels need to be raised;
(b) if a prolactin enhancer other than prolactin is administered, it will be administered during or some time shortly prior to the time interval when prolactin levels need to be raised (how much prior depends on pharmacokinetic properties: 0-3 hours prior has generally been found to be effective); and
(c) if a prolactin reducer is administered it will also be administered during or slightly prior to the time that prolactin levels need to be reduced (again, 0-3 hours prior has generally been found to be effective).

According to the present invention, "prolactin enhancer" includes prolactin as well as substances which increase circulating prolactin levels (e.g. by stimulating prolactin secretion). Non-limiting examples of a prolactin enhancer include prolactin; melatonin; dopamine antagonists such as haloperidol, pimozide, phenothiazine, domperidone, sulpiride and chlorpromazine; serotonin agonists, i.e., MAO-A inhibitors, e.g., synthetic morphine analogs, e.g., methadone; antiemetics, e.g., metoclopramide; estrogens; and various other serotonin agonists, e.g., tryptophan, 5-hydroxytryptophan (5-HTP), fluoxetine, and dexfenfluramine. Moreover, the non-toxic salts of the foregoing prolactin enhancing compounds formed from pharmaceutically acceptable acids are also useful in the practice of this invention. Melatonin and 5-HTP have been found particularly useful in the practice of this invention. Melatonin is particularly useful, because its administration at the appropriate time will also normalize abnormal melatonin rhythms, as shown below.

Nonlimiting examples of prolactin reducers include prolactin-inhibiting dopamine agonists (D2 agonists) such as dopamine and certain ergot-related prolactin-inhibiting compounds. Nonlimiting examples of dopamine agonists are 2-bromo-alpha-ergocriptine; 6-methyl-8-beta-carbobenzyloxy-aminoethyl-10-alpha-ergoline; 8-acylaminoergolines, are 6-methyl-8-alpha-(N-acyl)amino-9-ergoline and 6-methyl-8 alpha-(N-phenylacetyl)amino-9-ergoline; ergocornine; 9,10-dihydroergocornine; and D-2-halo-6-alkyl-8-substituted ergolines, e.g., D-2-bromo-6-methyl-8-cyanomethylergoline; carbi-dopa and L-dopa; and lisuride. Moreover, the non-toxic salts of the prolactin-reducer compounds formed with pharmaceutically acceptable acids are also useful in the practice of this invention. Bromocriptine, or 2-bromo-alpha-ergocryptine, has been found particularly useful in the practice of this invention.

The modulation of tumor growth inhibition induced by prolactin enhancers or reducers is expected to be dose-dependent over a range of dosages.

In treating mammals, generally, dosages of the prolactin reducer and/or enhancer, respectively, are each given, generally once a day, generally over a period ranging from about one month to about one year, but treatment can continue indefinitely (if necessary or desired) for months or even several years. The preferred prolactin reducer (accelerated release bromocriptine) is given at daily dosage levels ranging from about 3 micrograms to about 300 micrograms, preferably from about 10 micrograms to about 100 micrograms, per kg. of body weight, and a preferred prolactin enhancer. melatonin, is given at daily dosage levels ranging from about 10 micrograms to about 800 micrograms, preferably from about 10 micrograms to about 200 micrograms, per kg. of body weight per day to modify, or alter, the prolactin profile. Another preferred prolactin enhancer, 5-hydroxytryptophan, is given at daily dosage levels ranging from about 500 micrograms to about 13 milligrams per kg. of body weight, preferably from about 500 micrograms to about 2.5 milligrams per kg. of body weight. The exact dosage within these ranges to be administered to each subject will depend upon the particular prolactin modulator, the subject's age, stage of disease, physical condition and responsiveness to treatment.

In order to adjust the prolactin profile of a mammal, administration of a medicament comprising either one or both prolactin altering substances can be continued for a time sufficient to reset the circadian plasma prolactin rhythm to the phase and amplitude to that of a healthy subject of the same sex and species at which time treatment may be discontinued. If the subject suffers a relapse, treatment may be resumed in order to adjust the prolactin profile of the subject to conform or approach the prolactin profile of a healthy subject of the same sex and species. The time needed for resetting varies but is generally within the range of one month to one year. For some patients (e.g. patients in particularly poor physical condition, or those of an advanced age) it may not be possible to reset their prolactin rhythm within the above time periods and such patients may require a longer, or even continuous, treatment with medicaments comprising prolactin enhancers and/or reducers. The dosage and timing information set forth above is designed for bromocriptine, melatonin, and 5-hydroxytryptophan and will have to be altered for other agents using the dosage and timing methodology disclosed herein.

In the practice of this invention, a medicament comprising a prolactin reducing compound, and/or a prolactin enhancer is administered daily to a subject preferably orally, or by subcutaneous, intravenous or intramuscular injection. The reducer or enhancer can also be administered by inhalation. Dermal delivery systems e.g., skin patches, as well as suppositories and other well-known systems for administration of pharmaceutical agents can also be employed. Treatment generally lasts between about one month and about one year on average in humans. The administration of a medicament comprising prolactin reducer and/or prolactin enhancer in this manner will thus reset the phase and amplitude of the neural oscillators that control the body's ability to inhibit tumor growth to facilitate inhibition of tumor growth on a long term basis (e.g., several months or years). An improvement in the ability to inhibit tumor growth can be assessed by observation of partial or total ablation of the tumor or metastatic regrowth after the removal of a primary tumor. Instead of measuring tumor burden directly, well-known assays of tumor burden (e.g. assays of tumor-specific antigens, magnetic resonance imaging, CAT scanning, X-rays, ultrasound. counting blood-borne tumor cells in blood samples, etc.) can be used to assess the effect of treatment with timed administration of prolactin modulators.

Another approach to adjusting a cancer patient's abnormal profile is to follow these more specific guidelines to initially determine prolactin modulator administration timing, for a period of treatment of approximately 26 weeks for human subjects:
(i) Give prolactin reducers from 0600 hours to 1000 hours in a dosage range sufficient to decrease diurnal prolactin levels to within 1 SEM of the normal range of diurnal prolactin levels found in humans without tumors.
(ii) Give prolactin enhancers before or at bedtime in a dosage range sufficient to increase serum prolactin levels to at least the level of a normal, healthy human without tumors.

The aspect of the invention directed to an inhibition of tumor growth by resetting the prolactin profile of a mammalian subject (animal or human) having an aberrant prolactin profile with a medicament according to the present invention to conform to or approach the prolactin profiles for young healthy members of the same species and sex (e.g. the baselines of Figure 1) involves administration of a medicament comprising a prolactin reducer, or a prolactin enhancer, or both, at predetermined dosages and times dictated by the aberrant (pre-treatment) prolactin profile of the subject to be treated. The amounts of prolactin reducers and/or enhancers that are required to bring about this modification are within the same ranges as set forth above, but the time(s) of administration of these prolactin modulator(s) is determined by reference to how much and when the aberrant profile differs from the normal prolactin profile (baseline curve). Methods for determining the amounts and timing of administration are also set forth in copending U.S. patent application Serial No. 07/995,292 (now allowed) and its C-I-P, Ser. No. 08/264,558 filed June 23, 1994.

Another approach to normalizing the prolactin rhythm of a cancer patient by adjusting an abnormal prolactin profile is to give up to 4.8 mg/day of bromocriptine as follows; 0.8 mg/day for each of the first 7 days; beginning on day 8 and for 7 days thereafter, 1.6 mg/day is administered to the patient; beginning on day 15 and for 7 days thereafter, 2.4 mg/day are administered; beginning on day 22 and for 7 days thereafter, 3.2 mg/day is administered; beginning on day 29 and for 7 days thereafter, 4.0 mg/day is administered and beginning on day 36 and for 7 days thereafter, 4.8 mg per day is administered for 7 consecutive days. A preferred accelerated release bromocriptine dosage form has been disclosed in copending U.S. Patent Application Serial No. 08/171,897.

### DETERMINATION AND ADJUSTMENTS OF MELATONIN DAILY RHYTHMS

Healthy (normal) subjects, i.e., lean members of a species not suffering from tumors or any other pathologies have highly predictable daily melatonin profiles, which in humans have a characteristic sharp rise to a peak in the hours following the onset of sleep (23:00 to 4:00). "Healthy" individuals have melatonin profiles that are at within 1 SEM of the normal melatonin profile of Fig. 3, preferably for at least four melatonin levels measured at different times or within 2 SEM of the normal melatonin profile for at least two measured melatonin levels.

Normal daily melatonin profiles can be determined by methods identical to those described for prolactin profiles, except that blood samples are assayed for melatonin rather than prolactin. The daily melatonin profile of a tumor-bearing subject can also be determined by the methods described for prolactin, assaying blood samples for melatonin instead of prolactin.

Once a diurnal melatonin level profile has been developed for an individual. the profile is compared to the "normal" profile (e.g., the one generated as described in the previous section or to Fig. 3). A determination can then be made based on the following general criteria: from about 23:00 h till about 04:00 h, i.e., during the sleeptime peak of the normal daily melatonin profile, the individual's melatonin profile must first have a peak at about the same time or within two to six hours after sleep initiation as the "normal" melatonin peak for subjects in the same category (usually about 02:00-03:00) and must also be within one SEM of the normal healthy melatonin profile (preferably for four melatonin readings or alternatively within two SEM for at least two melatonin readings).

To determine if a subject has an aberrant melatonin profile, the bedtime on the subject's melatonin profile should ideally be coincident with the bedtime on the profile of normal subjects. If this is not the case, the profile of the subject and the profile of normal individuals can be superimposed and one or the other can be shifted so that the sleep initiation time of the subject to be tested coincides with the sleep initiation time of normal healthy subjects.

### Determination of Treatment for an Affected Subject

The information (melatonin profile or set of sleeptime melatonin levels) generated as described above is used to determine the type and extent of adjustment required. In general, those individuals that have tumors display abnormal melatonin profiles (or sleeptime melatonin levels) as compared to healthy individuals (compare, e.g., Figures 3 and 4). By adjusting the abnormal melatonin profile of such individuals by administration of a medicament comprising melatonin or a melatonin enhancer at the appropriate time of day and in the appropriate dosage (amount) it is possible to adjust such individuals' melatonin profile to conform (or at least approach) a normal profile. The amount and timing of administration of such dosages can be determined based upon information contained in the melatonin profiles (or sleeptime melatonin levels) discussed above, and based on the time it takes for the administered melatonin or melatonin enhancer to raise the melatonin levels in the subject's bloodstream.

An adjusted melatonin profile approaches a normal or healthy profile if all or a portion of the abnormal profile moves in the correct direction by at least 0.1 pmol/ml. For example, if a human subject's abnormal melatonin level is 0.01 pmol/ml between 24:00 and 01:00 and (after adjustment) it is increased to 0.11 pmol/ml during the same time period, the adjusted profile approaches the healthy profile. It is thus important to increase the area under the sleeptime melatonin curve (by at least about 30% and typically at least about 50%). It is also desirable not to exceed the normal sleeptime melatonin levels by more than 2 and preferably not more than 1 SEM (4 ng/ml and 2 ng/ml of plasma, respectively).

The treatment determination has two aspects: (a) timing of (each) dose of administration; and (b) amount of (each) dose to be administered.

Whether a full 24-hour or full night-time melatonin profile is generated for a subject to be treated, or only key sleeptime melatonin levels are measured, the following more specific guidelines will generally be followed to initially determine the timing of a medicament comprising melatonin, for a period of treatment of approximately 10 days to 26 weeks. A medicament comprising melatonin is administered once a day, at about bedtime. Generally, the daily dosage range by oral administration is from about 10 µg/kg to about 400 µg/kg of body weight; the preferred oral daily dosage is about 10 µg/kg to about 200 µg/kg of body weight. The preferred range is between about 40 µg/kg and 80 µg/kg of body weight. Melatonin is widely available commercially. The foregoing are applicable for setting initial therapy regimens.

The efficacy of a particular regimen on a particular patient and the adjustments (in dosage and timing) required, if any, can be determined by comparing the patient's re-evaluation melatonin profile or reevaluation sleeptime melatonin levels with the normal profile (or the "healthy" sleeptime profile levels).

Adjustments to the amount(s) of melatonin administered and possibly to the time of administration may be made as described above based on reevaluations.

The present timed daily treatment is typically continued over a period of time ranging from about 10 days to usually about 180 days, resulting in modification and resetting of the melatonin daily rhythm of the patient to that of a healthy person, at which time treatment may be discontinued. For some patients (e.g. patients in particularly poor physical condition, or those of an advanced age) it may not be possible to reset their melatonin rhythm within the above time periods and such patients may require a longer. or even continuous, treatment with a medicament comprising melatonin.

As indicated above, in the practice of the present invention normalization of the melatonin daily rhythm can be and preferably is practiced in conjunction with normalization of the prolactin daily rhythm.

### USE OF PHOTODYNAMIC THERAPY IN THE PRESENT INVENTION

### Preparation of the Photosensitizers

The preferred benzophenoxazine analogs for use in the present invention and the synthesis of the benzophenoxazine analogs are those described in Foley et al., U.S. Patent No. 4,962,197. The photosensitizer can be purified by medium pressure (100 psi) liquid chromatography using silica gel (Woelm 32-63) as a solid phase and eluting with a linear gradient of methylene chloride:methanol (100:0-90:10). The resulting purified photosensitizer is homogeneous by thin layer chromatography and high field nuclear magnetic resonance spectroscopy (JEOL 400 MHz). Aqueous solutions of the compound can be prepared in isotonic sucrose at a concentration of 0.175 mg/ml.

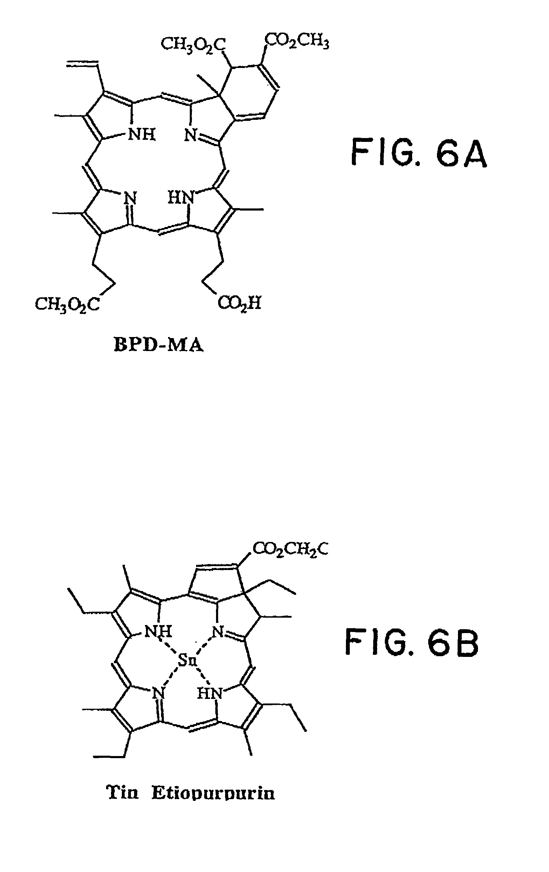

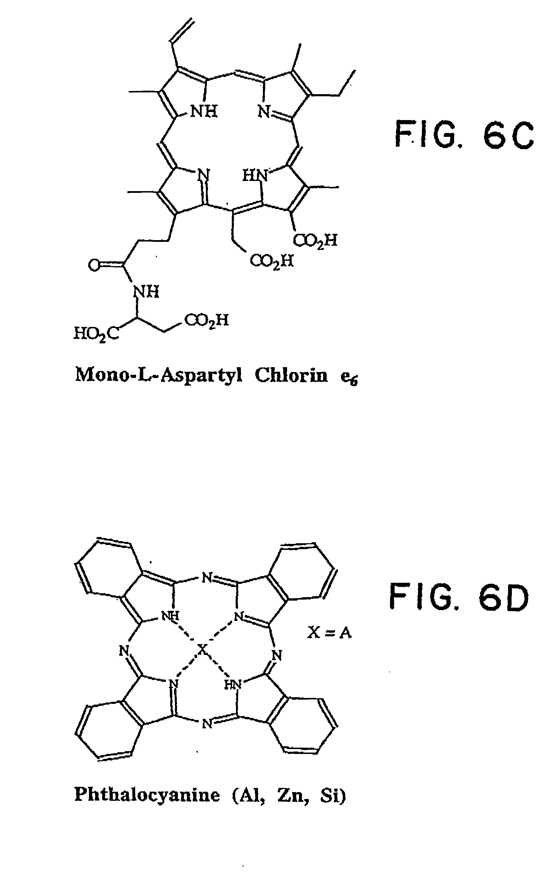

Benzoporphyrin derivative, mono acid ring a (BPD-MA) (Fig. 6) can be made by methods described in Levy et al., U.S. Patent No. 4,920,143 and Pahgka, V.S. et al., J. Org. Chem. 51:1094-1100, (1986). BPD-MA can also be obtained from Quadra Logic Technologies, Vancouver, BC, Canada. Methods for the synthesis of representative porphyrin photosensitizers to be used in the present invention can be found in Bommer, J.C. et al., European Patent Application No. 169,831; Shiau, F-Y. Et al., SPIE Institute Series IS6:71-86, 1990; Bonnet, R. Chemical Society Reviews 24:19-33, 1995; and Morgan, A.R. et al., Cancer Res. 48:194-198, 1988). Many of the porphyrin photosensitizers of the present invention are also commercially available. Monoaspartyl chlorin *e*6 (Fig. 6) can be obtained from Nippon Petrochemical, Tokyo, Japan. Purpurins. such as tin etiopurpurin (Fig. 6), can be obtained from PDT, Inc., Santa Barbara, CA. Bacteriochlorins, such as m-tetrahydrophenylchlorin (m-THPC), can be obtained from Scotia Pharmaceuticals, Guildford, England.

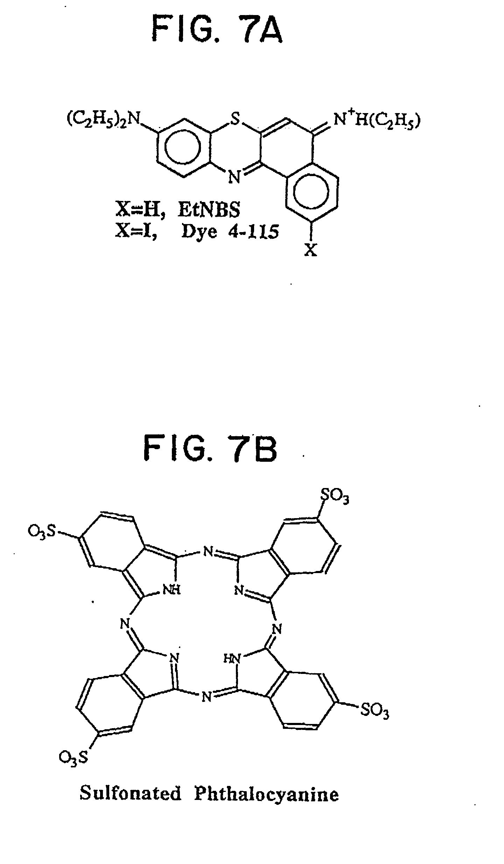

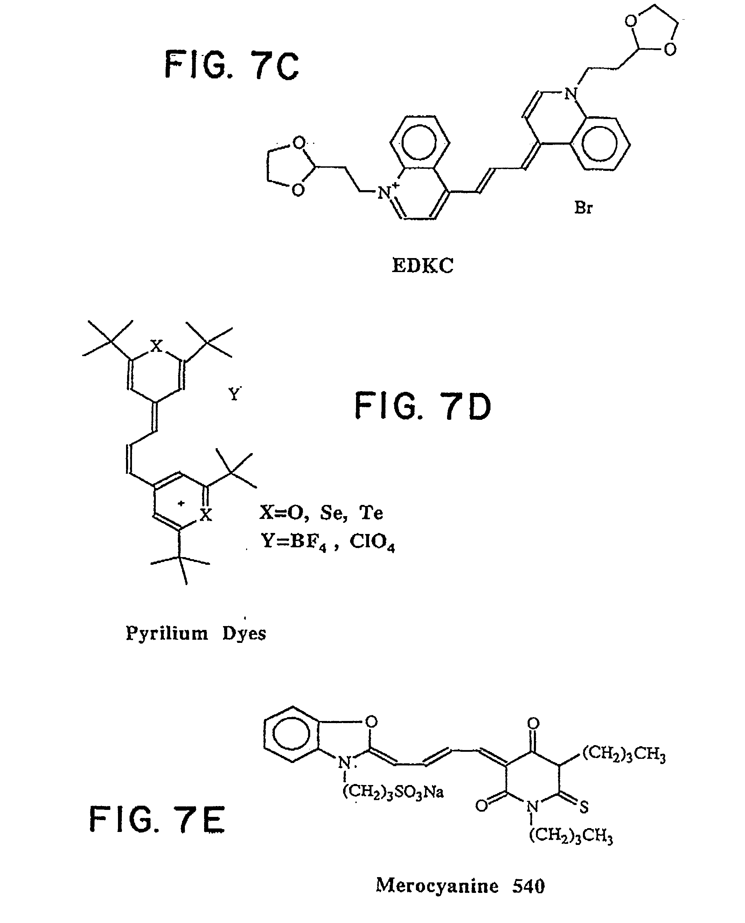

Methods for the synthesis of representative phthalocyanine photosensitizers (Fig. 6 and 7) to be used in the present invention can be found in Oleinick, N.L., et al., Photochem. Photobiol. 57:242-247, 1993; and Bonnet, R. Chemical Society Reviews 24:19-33, 1995. These compounds are also commercially available from Ciba Geigy, Basel, Switzerland, and Quadra Logic Technologies, Vancouver, BC, Canada.

N,N'-bis(2-ethyl-1,3-dioxolane)kryptocyanine (EDKC) (Fig. 7) can be prepared according to the method of Hamer in The Cyanine Dyes and Related Compounds (John Wiley & Sons, NY, 1964) and according to the method described in Oseroff et al., U.S. Patent 4,651,739. EDKC is also commercially available from Molecular Probes Inc., Eugene, OR. Merocyanines, such as Merocyanine 540 (Fig. 7) can be prepared according to methods described in Gunther, W.H.H., et al., Phosphorous, Sulfur, and Silicon 67:417-424, 1992; and methods for the synthesis of pyrilium dyes (Fig. 7) can be found in Detty, M.R., et al. Oncology Research 4:367-373, 1993.

The specific photosensitizers listed above are exemplary of the classes of benzophenoxazine analog, porphyrin, cyanine, and phthalocyanine dyes, and are not meant to limit the invention in any way to their sole use.

### Photosensitizer Dosage Forms and Administration

The benzophenoxazine analog is preferably a benzophenothiazine or pharmaceutically acceptable salt thereof. Most preferably, the photosensitizer is 5-ethylamino-9-diethylamino-2-iodobenzophenothiazinium chloride (Dye 4-115) (Fig. 7). The benzophenoxazine analog photosensitizer is typically dissolved in sterile isotonic sucrose or saline at 0.1 to 1.0 mg/ml, and preferably 0.25 mg/ml. Administration can be via an intravenous or subcutaneous route.

Administration of a medicament comprising a benzophenoxazine analog photosensitizer is generally such that between about 0.05 and about 10 mg/kg of body weight of photosensitizer is delivered to the patient, preferably between about 0.1 and about 5 mg/kg of body weight, and most preferably between about 0.5 and about 5 mg/kg of body weight. The active agent is preferably administered by infusion at between 0.1 and 0.5 ml per minute. With all the photosensitizers of the invention, it is preferred that a time interval passes between administration of photosensitizers and photoirradiation (i.e. exposure) of the tumor to light in order to give the photosensitizers time to reach the target tissues and to preferentially dissipate from normal cells, enhancing the differential photosensitizer concentration in tumor cells compared to normal cells. This time interval varies depending on the photosensitizer administered and the route of administration. When a benzophenoxazine analog such as Dye 4-115 is administered intravenously, the time interval is from between 0.5 and 5 hours, and preferably about 1 hour. When a benzophenoxazine analog such as Dye 4-115 is administered subcutaneously, the time interval is between about 0.5 and 5 hours, and preferably about 3 hours.

Administration of medicaments comprising other photosensitizers such as porphyrins, phthalocyanines, cyanines, and other benzophenoxazine analogs is carried out using techniques well-known to those of ordinary skill in the art for such chromophores.

### Light Activation of Administered Photosensitizers

Light-induced killing of solid tumors according to the invention can be carried out on any solid tumors which are accessible to light from conventional sources (e.g. a xenon arc lamp, an incandescent white light, a projector light source) or from a laser. If a tumor is on the body surface any light source including laser sources can be employed that provides light at the appropriate wavelengths to activate the dyes and that can deliver 50 to 200 mW per square centimeter of treated area. It is preferred to use a tunable argon-dye laser (a 5 watt argon ion pumped tunable dye laser, for example a Coherent, model Innova 100, Palo Alto, CA) using DCM (Exiton Chemical Co., Dayton, OH). Similar lasers are also commercially available from, for example, Spectra Physics, Mountain View, CA. However, a projector light source may also be employed. For tumors within the body, which are inaccessible to direct light sources, light is administered via optical fibers and the light source is a laser.

The light to which the tumor is exposed can be broadband white light containing wavelengths of between 600 and 900 nm. The light source must include light at the particular wavelength at which a given photosensitizer generates the most cytotoxin, e.g. singlet oxygen. Using filters, the broadband light can be narrowed to the specific wavelengths which excite particular photosensitizers. When a laser is used, it is tuned to the particular wavelength which most effectively excites a particular photosensitizer, generally the absorption maximum for a particular dye.

The absorption maxima of any particular dye can be determined by methods well-known in the art. Determination of absorption maxima is usually accomplished using a spectrophotometer.

Benzophenoxazine analogs have absorbance maxima at about 630-670 nm. BPD-MA has an absorbance maximum at about 690 nm. Mono-L-aspartyl chlorin *e*₆ has an absorption maximum at about 664 nm. Tin ethyl etiopurpurin has an absorbance maximum of about 666 nm. m-THPC has an absorbance maximum of about 652 nm. The phthalocyanines have absorption maxima at about 680 nm. EDKC has an absorption maxima at about 700 nm. Pyrilium dyes absorb maximally in about the 450-500 nm range. Merocyanine dyes have absorbance maxima from about 540 to about 626 nm.

In the practice of the invention, when using any of the photosensitizers of the invention, the total light energy delivered when irradiating tumors is between about 5 and about 400 Joules/cm², preferably about 100 Joules/cm². The power density of the light is preferably between about 50 and about 200 mWatts/cm², and is most preferably about 50 mWatts/cm². Delivery of laser light is carried out according to the well-known methods currently used for HPD-mediated laser therapy (Foultier et al., J. Photochem. Photobiol. B. Biol. 10:119-132, 1991). The output beam from the dye laser can be coupled to a quartz fiberoptic cable fitted with a microlens to ensure an even light distribution throughout the treatment field.

### COMBINED PHOTODYNAMIC AND NEUROENDOCRINE ADJUSTMENT TREATMENT OF TUMORS

The determination of the presence in a tumor bearing mammal of abnormal prolactin and melatonin rhythms and the adjustment of one or more of the abnormal rhythms to conform to or approach those of a healthy member of the same species and sex is undertaken as described above, comprising administering a medicament according to the present invention comprising prolactin reducers or enhancers, and/or melatonin enhancers, singly or in combination, at predetermined time intervals. During or after the hormonal rhythm adjustment treatment, photodynamic therapy is administered as described above. Preferably, photodynamic therapy is administered during the hormonal rhythm adjustment treatment. Most preferably, photodynamic therapy is administered about one to about two weeks subsequent to the initiation of hormonal rhythm adjustment treatment.

In mouse models, the typical response observed using PDT alone is that tumor "cure" (tumor-free for at least 90 days) of 4-8 mm diameter tumors can be achieved in a majority of the cases, largely dependent upon the tumor size at the time of PDT. Treatment of tumors consisting of the adjustment of only prolactin daily rhythms, as described in U.S. Patent application Serial No. 08/271,881 filed June 7, 1995, caused significant decreases in the growth of tumor tissue. Complete eradication of tumors, though, were not routinely achieved. It was unexpectedly discovered, however, that if the administration of a medicament comprising prolactin, at the appropriate time interval, was combined with PDT treatment then the tumor cure rate was close to 100%. This level of response has not been heretofore obtainable by using either PDT or prolactin resetting therapy alone. Thus a combination PDT and the adjustment of prolactin daily rhythms by a medicament according to the present invention show a synergistic effect in reducing the growth rate of or eradicating tumors. This synergistic effect is entirely unexpected, because there is no teaching in the prior art nor any reason for one of ordinary skill in the art to expect that prolactin would have any enhancing effects on the type I and type II photoreactions in which these dyes participate. Similarly, there is no teaching or suggestion in the prior art that would lead one to expect that timed administration of melatonin would act synergistically with PDT to kill tumors.

The present invention is further described and will be better understood by referring to the working Examples set forth below. These non-limiting Examples are to be considered illustrative only of the principles of the invention.

### EXAMPLE 1 Combination Photodynamic Therapy Plus Neuroendocrine Resetting Therapy to Inhibit Tumor Growth

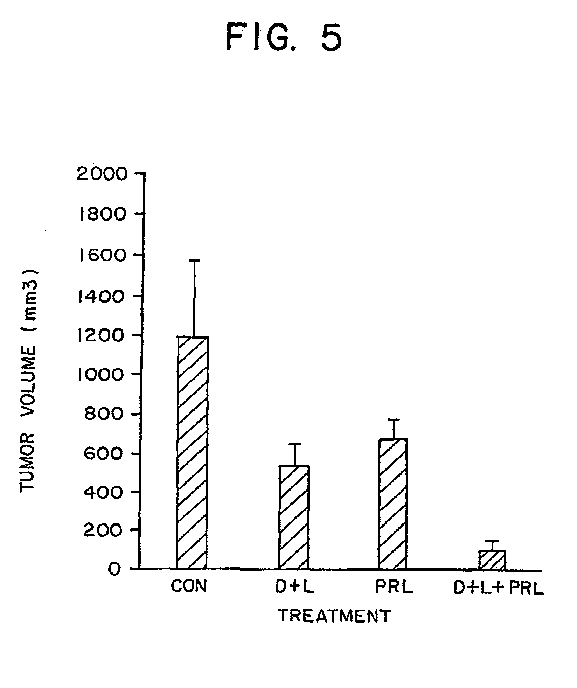

Adult (6-7 wk old) male Balb/c mice were subcutaneously injected with EMT-6 cells derived from a murine mammary sarcoma (1.7 x 10⁶ cells) on the hind quarter and were divided into four groups (n=10 per group) and treated as follows: Group 1 (D+L) received photodynamic therapy at 14 days after tumor inoculation. Photodynamic therapy consisted of subcutaneously injecting a benzophenothiazine photosensitizer (EtNBS, Figure 7)(7.5 mg/kg of body weight) and 3 hrs later irradiating the tumor with 652 nm light at a power density of 150 mW/cm2 and a total energy of 100J/cm². Group 2 (PRL) received intraperitoneal ovine prolactin (20 mcg/mouse) at 10 hours after light onset starting at 7 days after tumor inoculation and continuing for 14 days. Group 3 (D+L+PRL) received both PDT (as described above) and prolactin (as described above). Group 4 (CON) remained untreated (control). Fourteen days following PDT tumor volume was determined in all animals. The results are shown in Figure 5.

### EXAMPLE 2

Example 1 was repeated with different PDT power density and energy characteristics (for groups 2 and 3) which better optimize the PDT effect. The power density was changed to 50 mW/cm² and the total energy was increased to 180 J. Such changes have been shown to dramatically increase the PDT effect, theoretically by allowing for better reoxygenation of the tumor tissue which 1) increases the type 11 dependent PDT effect and 2) re-oxidizes reduced dye to the "active" form again, increasing the PDT effect. Under these conditions, tumor "cure" (tumor-free for at least 90 days) of 4-8 mm diameter tumors can be achieved in 70-100% of the cases largely dependent upon the tumor size at the time of PDT. However, if intraperitoneal prolactin administration (20 mcg/mouse/day at 10 hours after light onset) is added to PDT treatment (starting from the day of tumor cell inoculation), then the tumor cure rate is 100%.

The typical response observed using PDT alone with the PDT parameters employed are (in sequence): 1) an inflammation of the photoirradiated spot encompassing the tumor within about 30 minutes which increases to a maximum inflammation at about 3-5 hours; 2) inflammation completely subsides in 48-72 hours leaving noticeable tumor which takes 14 days to regress completely. Eschar formation occurs at 24-48 hours.

Contrariwise, when intraperitoneal prolactin injections are added to the PDT as described above, 100% of the treated animals exhibit severe eschar formation within 24 hours and complete tumor eradication occurs within this time frame. At 4-7 days the irradiated area has healed completely. This response cannot be obtained with PDT or prolactin alone.

### EXAMPLE 3

Adult (6-7 wk old) male Balb/c mice are subcutaneously injected with EMT-6 cells derived from a murine mammary sarcoma (1.7 x 10⁶ cells) on the hind quarter and are divided into four groups (n=10 per group) and are treated as follows: group 1 receives photodynamic therapy (PDT) at 14 days after tumor inoculation. PDT consists of intravenously injecting a benzophenothiazine photosensitizer (Dye 4-115)(2.5 mg/kg of body weight) and 1 hr later irradiating the tumor with a broad wavelength light source (600-700 nm) at a power density of 50 mW/cm² and a total energy of 100J/cm². Group 2 receives intraperitoneal ovine prolactin (20 mcg/mouse) at 10 hours after light onset starting at 7 days after tumor inoculation and continuing for 14 days. Group 3 receives both PDT (as described above) and prolactin (as described above). Group 4 remains untreated (control). Fourteen days following PDT tumor volume is determined in all animals.

The typical response which is observed using PDT alone with the PDT parameters employed are (in sequence): 1) an inflammation of the photoirradiated spot encompassing the tumor within about 30 minutes which increases to a maximum inflammation at about 3-5 hours; 2) inflammation completely subsides in 48-72 hours leaving noticeable tumor which takes 14 days to regress completely. Eschar formation occurs at 24-48 hours.

Contrariwise, when intraperitoneal prolactin injections are added to the PDT as described above, 100% of the treated animals will exhibit severe eschar formation within 24 hours and complete tumor eradication occurs within this time frame. At 4-7 days the irradiated area will heal completely. This response cannot be obtained with PDT or prolactin alone.

### EXAMPLE 4 Combination Photodynamic Therapy Plus Neuroendocrine Resetting Therapy with Bromocriptine to Inhibit Tumor Growth

Adult (6-7 wk old) male Balb/c mice are subcutaneously injected with EMT-6 cells (1.7 x 10⁶ cells) on the hind quarter and are divided into four groups (n=10 per group) and are treated as follows: group 1 receives photodynamic therapy (PDT) at 14 days after tumor inoculation. PDT consists of subcutaneously injecting a benzophenothiazine photosensitizer (EtNBS)(7.5 mg/kg of body weight) and 3 hrs later irradiating the tumor with 652 nm light at a power density of 50 mW/cm2 and a total energy of 100J/cm². Group 2 receives intraperitoneal bromocriptine (50 mcg/mouse) at 0 hours after light onset starting at 7 days after tumor inoculation and continuing for 14 days. Group 3 receives both PDT (as described above) and intraperitoneal bromocriptine (as described above). Group 4 remains untreated (control). Fourteen days following PDT tumor volume is determined in all animals. Tumors are found to be significantly reduced in the mice receiving either PDT treatment or intraperitoneal bromocriptine treatment, and are found to be significantly reduced or entirely eradicated in the mice receiving both therapies.

### EXAMPLE 5 Combination Photodynamic Therapy Plus Neuroendocrine Resetting Therapy with Melatonin to Inhibit Tumor Growth

Adult (6-7 wk old) male Balb/c mice are injected subcutaneously with EMT-6 cells (1.7 x 10⁶ cells) on the hind quarter and are divided into four groups (n=10 per group) and are treated as follows: group I receives photodynamic therapy (PDT) at 14 days after tumor inoculation. PDT consists of subcutaneously injecting a benzophenothiazine photosensitizer (EtNBS)(7.5 mg/kg of body weight) and 3 hrs later irradiating the tumor with 652 nm light at a power density of 50 mW/cm² and a total energy of 100J/cm². Group 2 receives intraperitoneal melatonin (8 mg/kg) at 10 hours after light onset starting at 7 days after tumor inoculation and continuing for 14 days. Group 3 receives both PDT (as described above) and intraperitoneal melatonin (as described above). Group 4 remains untreated (control). Fourteen days following PDT tumor volume is determined in all animals. Tumors are found to be significantly reduced in the mice receiving either PDT treatment or intraperitoneal melatonin treatment, and are found to be significantly reduced or entirely eradicated in the mice receiving both therapies.

### EXAMPLE 6 Combination Photodynamic Therapy Plus Neuroendocrine Resetting Therapy with Bromocriptine and Melatonin to Inhibit Tumor Growth

Adult (6-7 wk old) male Balb/c mice are subcutaneously injected with EMT-6 cells (1.7 x 10⁶ cells) on the hind quarter and are divided into four groups (n=10 per group) and are treated as follows: group 1 receives photodynamic therapy (PDT) at 14 days after tumor inoculation. PDT consists of subcutaneously injecting a benzophenothiazine photosensitizer (EtNBS)(7.5 mg/kg of body weight) and 3 hrs later irradiating the tumor with 652 nm light at a power density of 50 mW/cm² and a total energy of 100J/cm². Group 2 receives intraperitoneal melatonin (8 mg/kg) at 10 hours after light onset and intraperitoneal bromocriptine (50 mcg/mouse) at 0 hours after light onset starting at 7 days after tumor inoculation and continuing for 14 days. Group 3 receives both PDT (as described above) and intraperitoneal bromocriptine and intraperitoneal melatonin (as described above). Group 4 remains untreated (control). Fourteen days following PDT tumor volume is determined in all animals. Tumors are found to be significantly reduced in the mice receiving either PDT treatment or bromocriptine and melatonin treatment, and are found to be significantly reduced or entirely eradicated in the mice receiving both therapies.

## Claims

1. Use of a prolactin modulator and a photosensitizer for the preparation of a medicament for the simultaneous or sequential use of said prolactin modulator and said photosensitizer for treating a mammal bearing one or more tumors.

2. Use of a prolactin modulator for the preparation of a medicament or a pharmaceutical composition for the simultaneous or sequential use of said medicament or pharmaceutical composition with a photosensitizer for treating a mammal bearing one or more tumors.

3. Use of a photosensitizer for the preparation of a medicament or a pharmaceutical composition for the simultaneous or sequential use of said medicament or pharmaceutical composition with a prolactin modulator for treating a mammal bearing one or more tumors.

4. The use of claims 1-3, wherein said prolactin modulator is a prolactin enhancer.

5. The use of claims 1-3, wherein said prolactin modulator is a prolactin reducer.

6. The use of claim 4, wherein said prolactin enhancer is to be administered to said tumor bearing mammal at a predetermined time or times to increase the night time prolactin levels of said mammal so that said mammal's night time prolactin level conforms to or approaches the normal night time prolactin profile.

7. The use of any of claims 4 and 6, wherein said predetermined time is night time.

8. The use of any of claims 4, 6 and 7, wherein said prolactin enhancer is a member selected from the group consisting of prolactin, melatonin, metoclopramide, domperidone and 5-hydroxytryptophan.

9. The use of claim 8, wherein said prolactin enhancer is melatonin and said melatonin is to be administered in an amount within the range of 0.5-20 mg/person/day.

10. The use of claim 5, wherein said prolactin reducer is to be administered to said tumor bearing mammal at a predetermined time or times to decrease the day time prolactin levels of said mammal so that said mammal's day time prolactin level conforms to or approaches the normal day time prolactin profile.

11. The use of claims 5 and 10, wherein said predetermined time is between about 6:00h and 10:00h.

12. The use of claims 5, 10 and 11, wherein said prolactin reducer is a dopamine agonist.

13. The use of claim 12, wherein said prolactin reducer is bromocriptine and said bromocriptine is administered in an amount within the range of 0.8 to 4.8 mg/person/day.

14. The use of claims 1-13, wherein said tumor bearing mammal is a human.

15. The use of claims 1-14, wherein said photosensitizer is selected from the group consisting of porphyrin dyes, phtalocyanin dyes, cyanine dyes, benzophenoxazine analogs, and pharmaceutically acceptable salts thereof.

16. The use of claim 15, wherein said photosensitizer is a benzophenothiazine.

17. The use of claim 16, wherein said benzophenothiazine is a member selected from the group consisting of 5-ethylamino-9-diethylamino-benzo[a]phenothiazinium and 5-ethylamino-9-diethylamino-benzo-2-iodophenothiazinium, or a pharmaceutically acceptable salt thereof.

18. The use of claim 16, wherein said benzophenothiazine is a member selected from the group consisting of 5-ethylamino-9-diethylamino-benzo[a]phenothiazinium chloride and 5-ethylamino-9-diethylamino-benzo-2-iodophenothiazinium chloride (Dye 4-115).

## Patentansprüche

1. Verwendung eines Prolaktin-Modulators und eines Photosensitizers für die Herstellung eines Medikamentes für die gleichzeitige oder aufeinanderfolgende Verwendung des genannten Prolaktin-Modulators und des genannten Photosensitizers zur Behandlung eines Säugetiers, das an einem oder mehreren Tumoren leidet.

2. Verwendung eines Prolaktin-Modulators für die Herstellung eines Medikamentes oder einer pharmazeutischen Zusammensetzung für die gleichzeitige oder aufeinanderfolgende Verwendung des genannten Medikamentes oder der genannten pharmazeutischen Zusammensetzung mit einem Photosensitizer zur Behandlung eines Säugetiers, das an einem oder mehreren Tumoren leidet.

3. Verwendung eines Photosensitizers für die Herstellung eines Medikamentes oder einer pharmazeutischen Zusammensetzung für die gleichzeitige oder aufeinanderfolgende Verwendung des genannten Medikamentes oder der genannten pharmazeutischen Zusammensetzung mit einem Prolaktin-Modulator zur Behandlung eines Säugetiers, das an einem oder mehreren Tumoren leidet.

4. Die Verwendung gemäß den Ansprüchen 1-3, wobei der genannte Prolaktin-Modulator ein Prolaktin-Verstärker ist.

5. Die Verwendung gemäß den Ansprüchen 1-3, wobei der genannte Prolaktin-Modulator ein Prolaktin-reduzierendes Mittel ist.

6. Die Verwendung gemäß Anspruch 4, wobei der genannte Prolaktin-Verstärker dem genannten, an einem Tumor leidenden Säugetier zu einer vorherbestimmten Zeit oder zu vorherbestimmten Zeiten verabreicht werden soll, um die Prolaktin-Gehalte des genannten Säugetiers in der Nachtzeit so zu steigern, dass die Prolaktin-Gehalte des genannten Säugetiers in der Nachtzeit sich dem normalen Prolaktinprofil in der Nachtzeit anpaßt oder annähert.

7. Die Verwendung gemäß einem der Ansprüche 4 und 6, wobei die vorherbestimmte Zeit die Nachtzeit ist.

8. Die Verwendung gemäß einem der Ansprüche 4, 6 und 7, wobei der genannte Prolaktin-Verstärker ein Mitglied ist ausgewählt aus der Gruppe bestehend aus Prolaktin, Melatonin, Metoclopramid, Domperidon und 5-Hydroxytryptophan.

9. Die Verwendung gemäß Anspruch 8, wobei der genannte Prolaktin-Verstärker Melatonin ist und das genannte Melatonin in einer Menge innerhalb des Bereiches von 0,5-20 mg/Person/Tag verabreicht werden soll.

10. Die Verwendung gemäß Anspruch 5, wobei das genannte Prolaktinreduzierende Mittel dem genannten, an einem Tumor leidenden Säugetier zu einer vorherbestimmten Zeit oder zu vorherbestimmten Zeiten verabreicht werden soll, um die Prolaktin-Gehalte des genannten Säugetiers in der Tagzeit so zu reduzieren, dass die Prolaktin-Gehalte des genannten Säugetiers in der Tagzeit sich dem normalen Prolaktinprofil in der Tagzeit anpaßt oder annähert.

11. Die Verwendung gemäß den Ansprüchen 5 und 10, wobei die genannte vorherbestimmte Zeit etwa zwischen 6:00 Uhr und 10:00 Uhr liegt.

12. Die Verwendung gemäß den Ansprüchen 5, 10 und 11, wobei das genannte Prolaktin- reduzierende Mittel ein Dopamin Agonist ist.

13. Die Verwendung gemäß Anspruch 12, wobei das genannte Prolaktinreduzierende Mittel Bromocriptin ist und Bromocriptin in einer Menge im Bereich von etwa 0,8 bis 4,8 mg/Person/Tag verabreicht werden soll.

14. Die Verwendung gemäß den Ansprüchen 1-13, wobei das genannte an einem Tumor leidende Säugetier ein Mensch ist.

15. Die Verwendung gemäß Anspruch 1-14, wobei der genannte Photosensitizer aus einer Gruppe ausgewählt ist bestehend aus Porphyrinfarbstoffen, Phtalocyaninfarbstoffen, Cyaninfarbstoffen, Benzphenoxazinanaloga, und pharmazeutisch geeignete Salze davon.

16. Die Verwendung gemäß Anspruch 15, wobei der genannte Photosensitizer ein Benzophenothiazin ist.

17. Die Verwendung gemäß Anspruch 16, wobei das genannte Benzophenothiazin ein Mitglied aus der Gruppe ist bestehend aus 5-Ethylamino-9-diethylaminobenzo[a]phenothiazin und 5-Ethylamino-9-diethylamino-benzo-2-iodophenothiazin, oder einem pharmazeutisch geeignetem Salz davon.

18. Die Verwendung gemäß Anspruch 16, wobei das genannte Benzophenothiazin ein Mitglied aus der Gruppe ist bestehend aus 5-Ethylamino-9-diethylaminobenzo[a]phenothiazin-chlorid und 5-Ethylamino-9-diethylamino-benzo-2-iodophenothiazin-chlorid (Dye 4-115).

## Revendications

1. Utilisation d'un modulateur de la prolactine et d'un photosensibilisateur pour la préparation d'un médicament pour l'utilisation simultanée ou séquentielle dudit modulateur de la prolactine et dudit photosensibilisateur pour traiter un mammifère ayant une ou plusieurs tumeurs.

2. Utilisation d'un modulateur de la prolactine pour la préparation d'un médicament ou d'une composition pharmaceutique pour l'utilisation simultanée ou séquentielle dudit médicament ou de ladite composition pharmaceutique avec un photosensibilisateur pour traiter un mammifère ayant une ou plusieurs tumeurs.

3. Utilisation d'un photosensibilisateur pour la préparation d'un médicament ou d'une composition pharmaceutique pour l'utilisation simultanée ou séquentielle dudit médicament ou de ladite composition pharmaceutique avec un modulateur de la prolactine pour traiter un mammifère ayant une ou plusieurs tumeurs.

4. Utilisation selon l'une quelconque des revendications 1 à 3, dans laquelle ledit modulateur de la prolactine est un activateur de la prolactine.

5. Utilisation selon l'une quelconque des revendications 1 à 3, dans laquelle ledit modulateur de la prolactine est un réducteur de la prolactine.

6. Utilisation selon la revendication 4, dans laquelle ledit activateur de la prolactine doit être administré audit mammifère ayant une tumeur à un moment ou à des moments prédéterminé(s) afin d'augmenter les taux de prolactine la nuit dudit mammifère de telle sorte que le taux de prolactine la nuit dudit mammifère soit conforme à ou se rapproche du profil normal de la prolactine la nuit.

7. Utilisation selon l'une quelconque des revendications 4 et 6, dans laquelle ledit temps prédéterminé est la nuit.

8. Utilisation selon l'une quelconque des revendications 4, 6 et 7, dans laquelle ledit activateur de la prolactine est un élément choisi dans le groupe constitué de la prolactine, la mélatonine, le métoclopramide, le dompéridone et le 5-hydroxytryptophane.

9. Utilisation selon la revendication 8, dans laquelle ledit activateur de la prolactine est la mélatonine et ladite mélatonine doit être administrée en une quantité comprise dans la plage allant de 0,5 à 20 mg/personne/jour.

10. Utilisation selon la revendication 5, dans laquelle ledit réducteur de la prolactine doit être administré audit mammifère ayant une tumeur à un moment ou à des moments prédéterminé(s) afin de réduire les taux de prolactine le jour dudit mammifère de telle sorte que le taux de prolactine le jour dudit mammifère soit conforme à ou se rapproche du profil normal de la prolactine le jour.

11. Utilisation selon l'une quelconque des revendications 5 et 10, dans laquelle ledit temps prédéterminé est compris entre 6h00 et 10h00.

12. Utilisation selon l'une quelconque des revendications 5, 10 et 11, dans ledit réducteur de la prolactine est un agoniste de la dopamine.

13. Utilisation selon la revendication 12, dans ledit réducteur de la prolactine est la bromocriptine et ladite bromocriptine doit être administrée en une quantité comprise dans la plage allant de 0,8 à 4,8 mg/personne/jour.

14. Utilisation selon l'une quelconque des revendications 1 à 13, dans laquelle ledit mammifère ayant une tumeur est un être humain.

15. Utilisation selon l'une quelconque des revendications 1 à 14, dans laquelle ledit photosensibilisateur est choisi dans le groupe constitué des colorants de la porphyrine, des colorants de la phtalocyanine, des colorants de la cyanine, des analogues de la benzophénoxazine et de sels pharmaceutiquement acceptables de ceux-ci.

16. Utilisation selon la revendication 15, dans laquelle ledit photosensibilisateur est une benzophénothiazine.

17. Utilisation selon la revendication 16, dans laquelle ladite benzophénothiazine est un élément choisi dans le groupe constitué du 5-éthylamino-9-diéthylaminobenzo[a]phénothiazinium et du 5-éthylamino-9-diéthylamino-benzo-2-iodophénothiazinium ou d'un sel pharmaceutiquement acceptable de ceux-ci.

18. Utilisation selon la revendication 16, dans laquelle ladite benzophénothiazine est un élément choisi dans le groupe constitué du chlorure de 5-éthylamino-9-diéthylaminobenzo[a]phénothiazinium et du chlorure de 5-éthylamino-9-diéthylaminobenzo-2-iodophénothiazinium (colorant 4-115).
